# EUROPÄISCHE PATENTSCHRIFT

(11) **EP 3 612 833 B1**
(45) Veröffentlichungstag und Bekanntmachung des Hinweises auf die Patenterteilung: **07.06.2023**
(21) Anmeldenummer: 18717952.8
(22) Anmeldetag: 23.04.2018
(51) Int. Cl.: G01N 33/00

(54) **KOMPAKTES MESSGERÄT UND VERFAHREN ZUM ERFASSEN VON KOHLENWASSERSTOFFEN**
COMPACT MEASURING APPLIANCE AND METHOD FOR DETECTING HYDROCARBONS
APPAREIL DE MESURE COMPACT ET PROCÉDÉ POUR LA DETECTION D'HYDROCARBURES

(30) Priorität: 21.04.2017 DE 102017108609; 09.03.2018 WO PCT/EP2018/055927
(43) Veröffentlichungstag der Anmeldung: 26.02.2020
(73) Patentinhaber: Beko Technologies GmbH, 41468 Neuss (DE)
(72) Erfinder: PARUSEL, Franz-Josef, 40789 Monheim am Rhein (DE)
(74) Vertreter: Patentanwälte Bauer Vorberg Kayser
(86) Internationale Anmeldenummer: PCT/EP2018/060388
(87) Internationale Veröffentlichungsnummer: WO 2018/193138

(56) Entgegenhaltungen:
- EP-A1- 2 767 819
- WO-A1-2013/005332
- WO-A1-2015/170980
- DE-A1-102013 100 950
- US-A1- 2011 265 550
- US-A1- 2012 279 277

## Beschreibung

Die vorliegende Erfindung betrifft ein Messgerät und ein Verfahren zur Bestimmung einer Kohlenwasserstoff-Konzentration in einer Druckluft.

Derartige Messgeräte sind mit verschiedenen Sensortechnologien bekannt und dienen der Erfassung des Gehaltes von Öl, Kohlenwasserstoffen und oxidierbaren Gasen beispielsweise in Luft oder Druckluft. Häufig werden beispielsweise elektrisch beheizbare Halbleiter-Oxidmaterialien verwendet, die im beheizten Zustand ihren elektrischen Widerstand in Abhängigkeit von der Menge der in der Luft enthaltenen Kohlenwasserstoffe verändern.

Eine weitere Methode ist die Erfassung von Kohlenwasserstoffen mittels Elestoren. Dazu wird der zu messende Gasstrom über einen Körper aus beheiztem Katalysatormaterial geleitet, in dessen Inneren sich eine beheizte Platinwendel befindet. Die Kohlenwasserstoffkonzentration ist über die Änderung des elektrischen Widerstandes der beheizten und einer zweiten Platinwendel erfassbar, die sich durch die Verbrennungswärme des Kohlenwasserstoffanteils am Katalysator einstellt.

Ebenfalls bekannt ist die Verwendung von Flammenionisationsdetektoren. Bei derartigen Vorrichtungen werden die Kohlenwasserstoffe in einem Gasstrom verbrannt und die Spannungsänderung zwischen zwei Elektroden in der Flamme gemessen.

Eine weitere Methode ist die Erfassung der Kohlenwasserstoffkonzentration mittels Photoionisation. Dabei werden die Kohlenwasserstoffe mit ultraviolettem Licht bestrahlt. Die Energiemenge des Lichts muss dabei so hoch sein, dass Elektronen aus dem Kohlenwasserstoff herausgetrieben werden. Deren Anzahl lässt sich mit Elektroden messen.

Die oben genannten Verfahren eignen sich insbesondere für die Detektion höherer Konzentrationen in oxidierbaren Gasen, die Detektion geringerer Konzentrationen im unteren µg/Nm³-Bereich bzw. im ppb-Bereich ist aber nicht zuverlässig möglich.

Die mittels Photoionisationsdetektoren generierten Messwerte lassen nur indirekt auf die gemessene Stoffmenge schließen, da die Messwerte auch vom atomaren Aufbau der Verbindung abhängig sind und selbst bei gleichen Summenformeln recht stark variieren. Sofern die zu messende Verbindung aber konstant, bekannt und möglichst auch einheitlich ist, lässt sich die Konzentration des Kohlenwasserstoffanteils relativ zuverlässig messen. Allerdings sinkt die Messgenauigkeit mit abnehmender Konzentration an Kohlenwasserstoffen. Insbesondere steigt dabei der Einfluss des Feuchtegehalts der Luft. Mit abnehmendem Kohlenwasserstoffanteil wird der Einfluss der Luftfeuchte zunehmend größer, Messungen von Kohlenwasserstoffanteilen im unteren mg/Nm³-Bereich und insbesondere im µg/Nm³-Bereich sind nicht ausreichend genau durchzuführen.

Für die unterschiedlichen Anwendungen von Druckluft werden unterschiedliche Grenzwerte für den Ölanteil gefordert. Ölanteile bestehen aus tröpfchenförmigen Ölerosolen und aus Öldämpfen. Ölerosole und Öldämpfe können durch verschiedene Verfahren aus dem Druckluftstrom teilweise oder weitgehend eliminiert werden. Eine zeitnahe Messung von Öl in Druckluft ist aber bislang ein ungelöstes Problem.

Bekannte am Markt erhältliche Geräte sind oftmals sehr groß, oder sogar in zwei Gerätekomponenten, eine Sensoreinheit und einer Auswerteinheit unterteilt. Ein solches Gerät zeigt beispielsweise die DE 10 2013 100950 A1. Alternative Geräte und Messverfahren sind in den Druckschriften WO 2015 170 90 A1, EP 1 767 19 A1, WO 2013/005332 A1, WO 2011/265550 A1 und US 2012/279277 A1 beschrieben.

Die Aufgabe der Erfindung besteht darin, ein Messgerät zur Bestimmung einer Kohlenwasserstoff-Konzentration und ggf. oxidierbaren Gasen in Druckluft zu schaffen, dass zum einen geringste Konzentrationen zuverlässig misst und zum anderen die Nachteile des Standes der Technik nicht aufweist. Weiterhin ist es Aufgabe der Erfindung ein gegenüber dem Stand der Technik verbessertes Verfahren zur Bestimmung einer Kohlenwasserstoff-Konzentration und ggf. oxidierbaren Gasen in Druckluft bereit zu stellen.

Diese Aufgabe wird durch ein Messgerät mit den Merkmalen des Patentanspruchs 1 und durch ein Verfahren mit den Merkmalen des Patentanspruchs 10 gelöst.

Das erfindungsgemäße Messgerät zur Bestimmung einer Kohlenwasserstoff-Konzentration in einer Druckluft weist erfindungsgemäß ein einziges Gehäuse auf, in dem sämtliche erfindungswesentlichen Komponenten angeordnet sind.

Im Rahmen dieser Erfindung wird zwischen den folgenden Druckluftströmen unterschieden. Der Begriff Druckluft bezeichnet die in das Messgerät einströmende Kohlenwasserstoff-enthaltende Druckluft. Hierzu weist das Messgerät erfindungsgemäß eine Hauptgasleitung mit einem Einlass auf. Die Kohlenwasserstoff-enthaltende Druckluft kann direkt einer Sensoreinheit, sodann als zu messende Druckluft, zugeführt werden. Hierzu weist das Messgerät erfindungsgemäß eine von der Hauptgasleitung abgehende erste Gasleitung auf, wobei die erste Gasleitung die Hauptgasleitung mit einem ersten schaltbaren Ventil verbindet. Weiterhin weist das Messgerät erfindungsgemäß eine von der Hauptgasleitung abgehende zweite Gasleitung für eine Referenz-Druckluft auf. Als Referenz-Druckluft wird eine Druckluft bezeichnet, deren Kohlenwasserstoff-Konzentration unterhalb einer Nachweisgrenze liegt und daher im Sinne der vorliegenden Erfindung null beträgt. Erfindungsgemäß verbindet auch die zweite Gasleitung die Hauptgasleitung mit dem ersten schaltbaren Ventil. In der zweiten Gasleitung ist erfindungsgemäß eine Referenzgaseinheit angeordnet, die einen Oxidationskatalysator umfasst, der die Kohlenwasserstoffe zu Kohlendioxid und Wasser oxidiert. Die Referenz-Druckluft wird somit durch Abzweigen von der Kohlenwasserstoff-enthaltenden Druckluft aus der Hauptgasleitung der Referenzgaseinheit zugeführt. Erfindungsgemäß ist das erste schaltbare Ventil mit einer Sensoreinheit über eine dritte Gasleitung, die aus Kupfer besteht, verbunden.

Dadurch, dass die dritte Gasleitung aus Kupfer besteht, wird erreicht, dass die jeweilige, die dritte Gasleitung durchströmende, Druckluft die im Inneren des Gehäuses vorherrschende Temperatur annimmt bzw. sich auf diese erwärmt.

Die Sensoreinheit weist erfindungsgemäß einen Photoionisationsdetektor mit einer Messkammer auf, in der die Kohlenwasserstoff-Konzentration ermittelt werden kann. Das Messgerät führt die Kohlenwasserstoff-enthaltende Druckluft alternierend mittels des schaltbaren Ventils, vorzugsweise eines Magnetventils, direkt, als zu messende Druckluft, oder über die Referenzgaseinheit, als Referenz-Druckluft, zu der Sensoreinheit, die den Photoionisationsdetektor aufweist. Aus einer Signaldifferenz zwischen der zu messenden Druckluft und der Referenz-Druckluft wird ein Messwert ermittelt.

In einer alternativen Ausführungsvariante kann die Referenzgaseinheit auch Aktivkohle oder geeignete Membranen umfassen.

Das Messprinzip des Photoionisationsdetektors (PID) basiert vorliegend auf der Ionisation von in der Gasphase vorliegenden Kohlenwasserstoffen durch UV-Strahlung und der Erfassung des dabei entstehenden Ionenstroms. Die Stärke des Ionenstroms ist der Konzentration der ionisierten Kohlenwasserstoffe und ggf. weiterer ionisierbarer Gase direkt proportional. Das gemessene Signal kann ggf. elektronisch verstärkt als Konzentration der gemessenen Kohlenwasserstoffe ausgegeben werden.

Vorteilhafterweise zeigt der Photoionisationsdetektor (PID) die Gesamtkonzentration aller in der Probe enthaltenen, photoionisierbaren Verbindungen an und unterscheidet nicht zwischen einzelnen Komponenten bzw. Stoffen, so dass auch Kohlenwasserstoffverbindungen mit weniger als 6 Kohlenstoffatomen (<C6), wie beispielsweise Isobuten, detektierbar sind.

Die im Messgerät integrierte Referenzgaseinheit sichert nicht nur eine regelmäßige Bestimmung eines neuen Nullpunktes, sondern dient auch der regelmäßigen Reinigung des PID mittels der Kohlenwasserstofffreien, insbesondere öl- und fettfreien, Referenz-Druckluft.

Vorzugsweise wird dies durch eine strömungsoptimierte Geometrie der Messkammer unterstützt. Die Messkammer ist hierfür besonders bevorzugt trichterförmig mit einem sich in Strömungsrichtung verringernden Durchmesser ausgeführt. Dadurch wird ein Absetzen von Molekülen, Ölen und sonstigen Stoffen an der Innenwand der Messkammer wirksam reduziert. Zudem hat sich gezeigt, dass eine radial ausgerichtete Einströmung der jeweiligen Druckluft in die Messkammer aufgrund der sich daraus ergebenden Verweilzeit der Druckluft die Ionisation der Kohlenwasserstoffe und ggf. weiterer Gase unterstützt.

Als Oxidationskatalysator dient beispielsweise platinierte Quarzwolle, die in einem Behälter angeordnet ist.

Es hat sich gezeigt, dass eine, auf Grund der Oxidation in der Referenzgaseinheit, minimale Erhöhung der Feuchtigkeit dann vernachlässigbar ist, wenn bei der Messung Photoionisationslampen mit 10,6 eV verwendet werden, da bei solchen der Einfluss der Luftfeuchtigkeit am geringsten ist.

Weiterhin weist das erfindungsgemäße Messgerät einen in der Hauptgasleitung angeordneten Druckregler zur Sicherstellung eines konstanten Durchflusses der Druckluft im Bereich von 3 bis 16 bar auf, so dass immer gleiche Betriebsbedingungen für die Sensoreinheit, insbesondere den PID, sichergestellt werden können, was wiederum die Messgenauigkeit erhöht.

Die ermittelten Messwerte werden für eine besonders genaue Messung temperatur- und druckkompensiert. Hierzu weist das Messgerät erfindungsgemäß ein stromaufwärts der Sensoreinheit angeordnetes Druckmessgerät zur Bestimmung eines Druckes der jeweiligen, in die Sensoreinheit einströmenden, Druckluft, sowie einen stromabwärts der Sensoreinheit angeordneten Temperaturfühler zur Bestimmung einer Temperatur der jeweiligen, die Sensoreinheit verlassenden, Druckluft. Der ermittelte Messwert bezieht sich der somit stets auf eine vorgeschriebene Normtemperatur von 20 °C und einen vorgeschriebenen Luftdruck von 1000 mbar.

Zur Kühlung der innerhalb des Messgeräts angeordneten Referenzgaseinheit weist das Messgerät erfindungsgemäß eine Kühlvorrichtung auf. So kann die im Betrieb entstehende Wärme wirkungsvoll abgeführt werden. Erfindungsgemäß ist die Kühlvorrichtung als Lüfter ausgeführt, so dass Umgebungsluft in das Gehäuse einströmt und erwärmte Luft aus dem Inneren des Gehäuses ausströmt.

In einer besonders vorteilhaften Ausführungsvariante wird für eine entsprechende Betriebssicherheit die Funktion der Referenzgaseinheit und der Sensoreinheit, insbesondere des PID dauerhaft überwacht und durch ein Anzeigemittel, vorzugsweise eine LED signalisiert. Bei Unter- bzw. Überschreitung eines definierten Sicherheitsgrenzwertes wird ein Alarm aktiviert und der Anwender erhält den Hinweis, dass eine Überprüfung des Gerätes notwendig ist. Hierzu schaltet, im Fall einer Funktionsstörung, beispielsweise die LED von grün auf rot um. Der Durchfluss zur Sensoreinheit, insbesondere zum PID, wird unterbrochen, wodurch der Photoionisationsdetektor vor übermäßigen Belastungen geschützt wird.

Vorzugsweise weist das Messgerät hierzu ein in der dritten Gasleitung stromabwärts des ersten schaltbaren Ventils angeordnetes zweites schaltbare Ventil, insbesondere ein Magnetventil, sowie einen zwischen dem zweiten Ventil und dem Druckmessgerät angeordneten Druckschalter auf.

In einer weiteren vorteilhaften Ausführungsvariante weist das Messgerät stromabwärts des Druckreglers ein in der Hauptgasleitung angeordnetes Sicherheitsventil auf, das bei einem Druck ≥ 4 bar öffnet.

Vorzugsweise ist in der Hauptgasleitung stromabwärts des Druckreglers ein weiteres Druckmessgerät, beispielsweise ein Manometer, zur Überwachung des Druckes der Druckluft, angeordnet.

Weiterhin weist das Messgerät vorzugsweise eine integrierte Auswerteeinheit auf, die eine Auswerteelektronik und eine Bedienoberfläche (Display) umfasst. Die Bedienoberfläche kann gleichzeitig als Eingabeeinheit ausgeführt sein, beispielsweise durch einen Touchscreen.

Nachfolgend wird genauer auf die weiteren Aspekte der vorliegenden Erfindung eingegangen. Zur Vermeidung von Wiederholungen wird auf die vorstehende Diskussion der Merkmale und Vorteile der diversen Ausgestaltungen des erfindungsgemäßen Messgeräts verwiesen, die unmittelbar auf die nachfolgende Diskussion der verschiedenen Ausgestaltungen und Weiterbildungen übertragbar sind.

In einem weiteren Aspekt betrifft die vorliegende Erfindung ein Verfahren zur Bestimmung einer Kohlenwasserstoff-Konzentration in einer Druckluft mittels des erfindungsgemäßen Messgeräts, aufweisend die Verfahrensschritte:
- Einleiten der Kohlenwasserstoff-enthaltenden Druckluft in das Messgerät und einstellen eines konstanten Durchflusses in einem Druckbereich von 3 bis 16 bar,
- Aufteilen der Kohlenwasserstoff-enthaltenden Druckluft in eine zu messende Druckluft und eine Referenz-Druckluft, wobei die zu messende Druckluft direkt einer Sensoreinheit zugeführt und die Referenz-Druckluft zunächst oxidiert wird,
- Einleiten der zu messenden Druckluft und der Referenz-Druckluft im Wechsel in die Sensoreinheit in einem festgelegten Intervall und bestimmen eines Druckes der jeweiligen, in die Sensoreinheit einströmenden, Druckluft,
- Ermitteln eines Messwerts aus einer Signaldifferenz zwischen der zu messenden Druckluft und Referenz-Druckluft, und
- Bestimmen einer Temperatur der jeweiligen, die Sensoreinheit verlassenden, Druckluft.

Erfindungsgemäß wird die Kohlenwasserstoff-enthaltende Druckluft kontinuierlich in die zu messende Druckluft und Referenz-Druckluft unterteilt, wobei die die Referenz-Druckluft der Referenzgaseinheit ebenfalls kontinuierlich zugeführt wird. Sofern keine Nullluft bzw. keine Referenz-Druckluft benötigt wird, wird diese über einen stromabwärts der Referenzeinheit angeordneten Schalldämpfer an die Umwelt abgeführt. Schaltet das Messgerät auf die Messung von Referenz-Druckluft um, steht dieses unmittelbar zur Verfügung und kann ohne Zeitverlust verwendet werden. Ein, wie im Stand der Technik, erforderliches Hochfahren der Referenzgaseinheit ist somit nicht notwendig.

Die Kalibrierung des PID mittels der Referenz-Druckluft erfolgt erfindungsgemäß nach einem festgelegten Intervall, vorzugsweise wird die zu messende Druckluft um einen Faktor im Bereich von 1,5 bis 5, vorzugsweise 2 bis 4, länger der Sensoreinheit zugeführt, als die Referenz-Druckluft.

Beispielsweise wird der Sensoreinheit im Wechsel für sechs Minuten die zu messende Druckluft und anschließend für zwei Minuten die Referenz-Druckluft zugeführt. Durch den automatischen Nullpunkt-Abgleich werden die üblichen Drifteffekte kompensiert und somit, auch bei einem niedrigen Kohlenwasserstoffgehalt, insbesondere Öl, beispielsweise von kleiner 0,01 mg/m³, hochgenaue Messwerte ermittelt. Der Messbereich beträgt vorzugsweise kleiner 2,5 mg/m³, besonders bevorzugt im Bereich von 0,01 bis 2,5 mg/m³.

Der ermittelte Messwert ist eine Spannungsdifferenz zwischen der letzten gemessenen Null-Spannung auf Basis der Referenz-Druckluft und der aktuell gemessenen Signalspannung der zu messenden Druckluft. Diese Spannungsdifferenz kann vorzugsweise mit hinterlegten Referenzwerten verglichen werden, um eine Umrechnung in eine Konzentration in ppb zu ermöglichen.

Um die Signalspannung des PID in einem Prozessor weiterverarbeiten zu können, muss sie zunächst digitalisiert werden. Dazu wird ein Schaltkreis eingesetzt, der als Analog - Digitalwandler bezeichnet wird. Das Resultat ist eine sehr genau gemessene Spannung, die dem Prozessor in bestimmten Abständen zur Verfügung gestellt wird. Zur genauesten möglichen Umwandlung benötigt der Schaltkreis mindestens 0,4 Sekunden.

Da das Messergebnis jedoch nicht in diesen kurzen Intervallen benötigt wird, wird vorzugsweise aus einer Vielzahl von, besonders bevorzugt 5 bis 15, ermittelten Messwerten ein Mittelwert gebildet wird.

Da sehr geringe Spannungen gemessen werden, besteht die Gefahr, dass Messfehler bei jeder einzelnen Messung auftreten können. Durch die Zusammenfassung von mehreren Messwerten, beispielsweise 10, ist der Messfehler bei einem konstanten Signal 3-mal kleiner als bei der Einzelmessung, d.h. die Messung über 4 Sekunden ist somit also etwa 3-mal genauer als ein einzelner Messwert.

Je nach Anwendungsfall können auch mehr oder weniger Messwerte für die Mittelwertbildung herangezogen werden.

Es hat sich gezeigt, dass anstelle einer Mittelwertbildung die Ermittlung eines gleitenden Mittelwerts zu besseren Ergebnissen führt. Dabei werden neben dem aktuellen Mittelwert auch vorangegangene Mittelwerte berücksichtigt. Insgesamt wird der Mittelwert also jeweils aus dem aktuellen und unmittelbar vorangegangene Mittelwerten ermittelt, wobei die Anzahl einbezogener vorangegangener Mittelwerte je nach Anwendungsfall variierbar ist.

Durch dieses vorteilhafte Berechnungsverfahren werden einzelne Fehlmessungen, die etwa durch Störungen im Stromnetz verursacht werden können, wirkungsvoll unterdrückt.

Vorzugsweise wird der gleitende Mittelwert aus einer Vielzahl von Mittelwerten, besonders bevorzugt aus 15 bis 25, gebildet.

Die Erfindung wird im Folgenden mit Bezug auf die beiliegenden Figuren näher erläutert. Dabei zeigen die Figuren lediglich eine vorteilhafte Ausführungsvariante, die Erfindung soll aber keinesfalls auf diese beschränkt sein, sondern nur auf die beigefügte Ansprüche. Es zeigen:
- Fig. 1:: eine Prinzipdarstellung eines Innenraums einer Ausführungsvariante des erfindungsgemäßen Messgerätes, und
- Fig. 2:: ein Funktionsschema, bzw. Schaltbild der Fluidströme des Messgeräts.

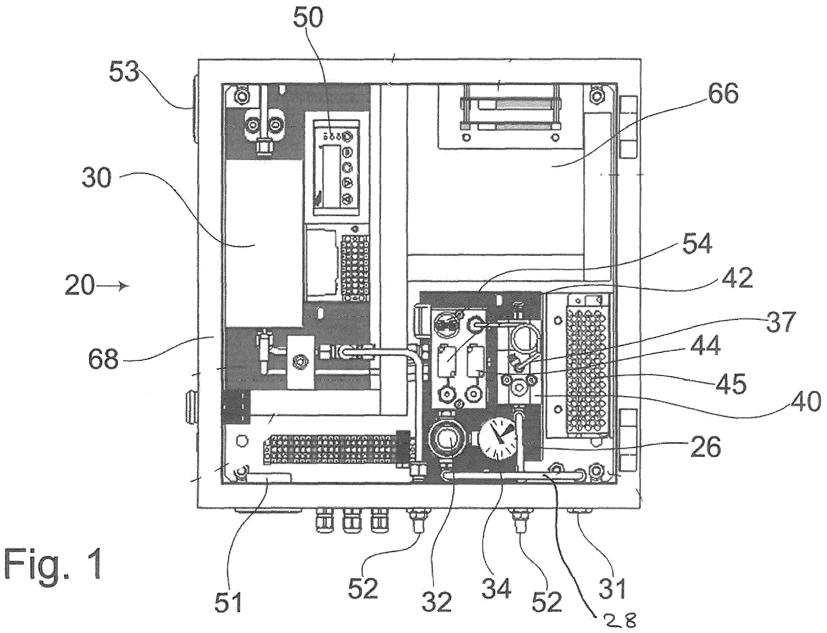

Figur 1 zeigt einen Innenraum einer Ausführungsvariante des erfindungsgemäßen Messgeräts 20 zur Bestimmung einer Kohlenwasserstoff-Konzentration in einer Druckluft mit sämtlichen in einem einzigen Gehäuse 68 angeordnete Komponenten.

Das Messgerät 20 weist eine Hauptgasleitung 28 mit einem Einlass 31 für die Druckluft auf. Von der Hauptgasleitung 28 geht eine erste Gasleitung 38 für eine zu messende Druckluft ab, wobei die erste Gasleitung 38 die Hauptgasleitung 28 mit einem ersten schaltbaren Ventil 42 verbindet. Weiterhin geht von der Hauptgasleitung 28 eine zweite Gasleitung 46 für eine Referenz-Druckluft ab, wobei auch die zweite Gasleitung 46 die Hauptgasleitung 28 mit dem ersten schaltbaren Ventil 42 verbindet. Weiterhin weist das Messgerät eine das erste schaltbare Ventil 42 mit einer Sensoreinheit 26 verbindende dritte Gasleitung 37 auf, die aus Kupfer besteht.

Die Sensoreinheit 26 weist einen Photoionisationsdetektor 40 mit einer Messkammer (nicht dargestellt) auf, der die jeweilige Druckluft zugeführt wird.

Weiterhin ist in der zweiten Gasleitung 46 eine Referenzgaseinheit 30 enthaltend einen Oxidationskatalysator (nicht dargestellt) angeordnet. In der Referenzgaseinheit 30 wird aus der kohlenwasserstoff-enthaltenden Druckluft die Referenz-Druckluft, also eine Kohlenwasserstofffreie Druckluft, erzeugt.

In der Hauptgasleitung 28 ist ferner ein Druckregler 32 zur Sicherstellung eines konstanten Durchflusses der Druckluft im Bereich von 3 bis 16 bar angeordnet.

Zur Bestimmung eines Druckes und einer Temperatur der jeweiligen, in die Sensoreinheit 26 einströmenden bzw. aus der Sensoreinheit 26 ausströmenden, Druckluft weist das Messgerät 20 ein stromaufwärts der Sensoreinheit 26 angeordnetes Druckmessgerät 56 sowie einen stromabwärts der Sensoreinheit 26 angeordneten Temperaturfühler 62 auf.

Weiterhin weist das Messgerät eine Kühlvorrichtung 51, 53 zur Kühlung der Referenzgaseinheit 30 auf.

Stromabwärts des ersten schaltbaren Ventils 42 ist in der dritten Gasleitung 37 ein zweites schaltbare Ventil 44 sowie stromabwärts hierzu ein Druckschalter 54 angeordnet.

Die Referenzgaseinheit 30 weist weiterhin einen Temperaturregler 50 mit Alarmfunktion sowie zur Kühlung eine Kühlvorrichtung aus einem Lüfter 51 sowie einem Luftaustritt 53 aus.

Über Schalldämpfer 52 kann die zu messende Druckluft bzw. die Referenz-Druckluft das Messgerät 20 verlassen.

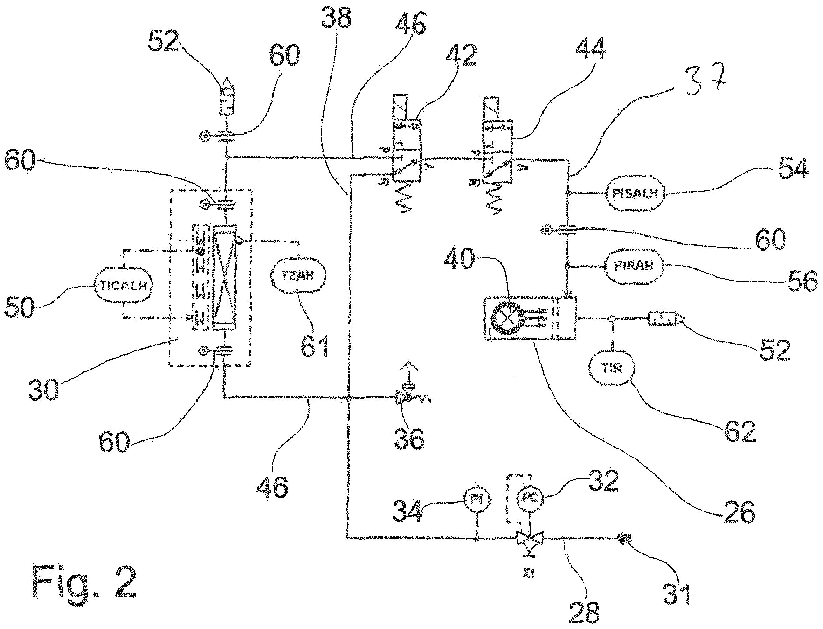

Aus der Figur 2 wird die Funktionsweise des erfindungsgemäßen Messgeräts 20 verdeutlicht. Durch den Einlass 31 wird die Kohlenwasserstoff-enthaltende Druckluft in das Messgerät 20 eingeleitet. Der Druckregler 32 regelt den Einströmdruck. Stromabwärts des Druckreglers 32 folgt das Sicherheitsventil 36, das beispielsweise bei einem Druck ≥ 4 bar öffnet.

Von der Kohlenwasserstoff-enthaltenden Druckluft innerhalb der Hauptgasleitung 28 wird die zu messende Druckluft über die erste Gasleitung 38 der Sensoreinheit 26 bzw. dem Photoionisationsdetektor 40 (PID) über das erste schaltbare Ventil 42 und das zweite schaltbare Ventil 44 direkt zugeführt.

Eine Referenz-Druckluft wird über eine zweite Gasleitung 46 zunächst der Referenzgaseinheit 30 zugeführt, bevor diese über die beiden Ventile 42, 44 ebenfalls dem PID 40 zugeführt wird. Sofern keine Referenz-Druckluft erforderlich ist, wird diese über einen Schalldämpfer 52 an die Umwelt abgeführt.

Die Referenzgaseinheit 30 weist den Temperaturregler 50 auf. Übersteigt die Temperatur der Referenz-Druckluft einen Grenzwert, wird das zweite schaltbare Ventil 44 geschlossen und über eine Alarmvorrichtung 61 ein Alarm ausgegeben.

Stromabwärts der beiden schaltbaren Ventile 42, 44 ist der elektromechanischer Druckschalter 54 angeordnet, der das zweite schaltbare Ventil 44 bei Über- oder Unterdruck schließt und einen Alarm auslöst.

Der Temperaturfühler 62 überwacht die Temperatur der jeweiligen Druckluft in dem Sensoreinheit 26 und gibt bei Übersteigen eines Grenzwerts ein Alarmsignal aus.

Innerhalb des Messgeräts 20 sind weiterhin Drosseln 60 zur Regelung des Durchflusses der jeweiligen Druckluft vorgesehen. Ferner umfasst das Messgerät 20 ein Netzteil 45 sowie einen Prozessor mit zugehöriger Platine etc..

## Patentansprüche

1. Messgerät (20) zur Bestimmung einer Kohlenwasserstoff-Konzentration in einer Druckluft aufweisend ein Gehäuse (68) und folgende im Gehäuse (68) angeordnete Komponenten:
- eine Hauptgasleitung (28) mit einem Einlass (31) für die Druckluft,
- eine von der Hauptgasleitung (28) abgehende erste Gasleitung (38) für eine zu messende Druckluft, wobei die erste Gasleitung (38) die Hauptgasleitung (28) mit einem ersten schaltbaren Ventil (42) verbindet,
- eine von der Hauptgasleitung (28) abgehende zweite Gasleitung (46) für eine Referenz-Druckluft, wobei die zweite Gasleitung (46) die Hauptgasleitung (28) mit dem ersten schaltbaren Ventil (42) verbindet,
- eine das erste schaltbare Ventil (42) mit einer Sensoreinheit (26) verbindende dritte Gasleitung (37) bestehend aus Kupfer, so dass die dritte Gasleitung (37) durchströmende Druckluft die im Inneren des Gehäuses (68) vorherrschende Temperatur annimmt, wobei die Sensoreinheit (26) einen Photoionisationsdetektor (40) mit einer Messkammer aufweist,
- eine in der zweiten Gasleitung (46) angeordnete Referenzgaseinheit (30) enthaltend einen Oxidationskatalysator in der aus der Druckluft die Referenz-Druckluft erzeugt wird,
- einen in der Hauptgasleitung (28) angeordneten Druckregler (32) zur Sicherstellung eines konstanten Durchflusses der Druckluft im Bereich von 3 bis 16 bar,
- ein stromaufwärts der Sensoreinheit (26) angeordnetes Druckmessgerät (56) zur Bestimmung eines Druckes der jeweiligen, in die Sensoreinheit (26) einströmenden, Druckluft,
- einen stromabwärts der Sensoreinheit (26) angeordneten Temperaturfühler (62) zur Bestimmung einer Temperatur der jeweiligen, die Sensoreinheit (26) verlassenden, Druckluft, und
- eine Kühlvorrichtung (51, 53) zur Kühlung der Referenzgaseinheit (30), wobei die Kühlvorrichtung (51, 53) zur Kühlung der Referenzgaseinheit (30) als Lüfter ausgeführt ist, so dass Umgebungsluft in das Gehäuse (68) einströmt und erwärmte Luft aus dem Inneren des Gehäuses (68) ausströmt.

2. Messgerät (20) nach Anspruch 1, aufweisend ein in der dritten Gasleitung (37) stromabwärts des ersten schaltbaren Ventils (42) angeordnetes zweites schaltbare Ventil (44).

3. Messgerät (20) nach Anspruch 2, wobei das erste und zweite schaltbare Ventil (42, 44) ein Magnetventil ist.

4. Messgerät (20) nach einem der vorhergehenden Ansprüche 2 oder 3, aufweisend ein in der dritten Gasleitung (37) zwischen dem zweiten Ventil (44) und dem Druckmessgerät (56) angeordneten Druckschalter (54).

5. Messgerät (20) nach Anspruch 1, wobei die Messkammer trichterförmig ausgebildet ist.

6. Messgerät (20) nach Anspruch 1, wobei das Messgerät (20) weiterhin eine Auswerteeinheit umfasst.

7. Messgerät (20) nach Anspruch 1, aufweisend ein in der Hauptgasleitung (28) stromabwärts des Druckreglers (32) angeordnetes weiteres Druckmessgerät (34).

8. Messgerät (20) nach Anspruch 1, aufweisend ein in der Hauptgasleitung (28) stromabwärts des Druckreglers (32), vorzugsweise des weiteren Druckmessgeräts (34), angeordnetes Sicherheitsventil (36).

9. Verfahren zur Bestimmung einer Kohlenwasserstoff-Konzentration in einer Druckluft mittels eines Messgeräts (20) nach einem der vorhergehenden Ansprüche 1 bis 8, aufweisend die Verfahrensschritte:
- Einleiten der Kohlenwasserstoff-enthaltenden Druckluft in das Messgerät (20) und Einstellen eines konstanten Durchflusses in einem Druckbereich von 3 bis 16 bar,
- Aufteilen der Kohlenwasserstoff-enthaltenden Druckluft in eine zu messende Druckluft und eine Referenz-Druckluft, wobei die zu messende Druckluft direkt einer Sensoreinheit (26) zugeführt und die Referenz-Druckluft zunächst oxidiert wird,
- Einleiten der zu messenden Druckluft und der Referenz-Druckluft im Wechsel in die Sensoreinheit (26) in einem festgelegten Intervall und bestimmen eines Druckes der jeweiligen, in die Sensoreinheit (26) einströmenden, Druckluft,
- Ermitteln eines Messwerts aus einer Signaldifferenz zwischen der zu messenden Druckluft und Referenz-Druckluft, und
- Bestimmen einer Temperatur der jeweiligen, die Sensoreinheit (26) verlassenden, Druckluft.

10. Verfahren nach Anspruch 9, wobei die zu messende Druckluft um einen Faktor im Bereich von 1,5 bis 5, vorzugsweise 2 bis 4, länger der Sensoreinheit (26) zugeführt wird.

11. Verfahren nach Anspruch 9 oder 10, wobei aus einer Vielzahl von, vorzugsweise 5 bis 15, ermittelten Messwerten ein Mittelwert gebildet wird.

12. Verfahren nach Anspruch 11, wobei aus einer Vielzahl von Mittelwerten, vorzugsweise aus 15 bis 25, ein gleitender Mittelwert gebildet wird.

## Claims

1. A measuring device (20) for determining a hydrocarbon concentration in compressed air, comprising a housing (68) and the following components disposed in the housing (68):
- a main gas line (28) with an inlet (31) for the compressed air,
- a first gas line (38) for a compressed air to be measured branching off from the main gas line (28), wherein the first gas line (38) connects the main gas line (28) to a first switchable valve (42),
- a second gas line (46) for a reference compressed air branching off from the main gas line (28), wherein the second gas line (46) connects the main gas line (28) to the first switchable valve (42),
- a third gas line (37) made from copper connecting the first switchable valve (42) to a sensor unit (26), so that compressed air flowing through the third gas line (37) assumes the temperature prevailing in the interior of the housing (68), wherein the sensor unit (26) has a photoionization detector (40) with a measurement chamber,
- a reference gas unit (30) arranged in the second gas line (46), containing an oxidation catalyst, in which the reference compressed air is generated from the compressed air,
- a pressure regulator (32) arranged in the main gas line (28) for ensuring a constant through-flow of compressed air in the range from 3 to 16 bar,
- a pressure measuring device (56) arranged upstream of the sensor unit (26) for determining a pressure of the respective compressed air flowing into the sensor unit (26),
- a temperature sensor (62) arranged downstream of the sensor unit (26) for determining a temperature of the respective compressed air leaving the sensor unit (26), and
- a cooling device (51, 53) for cooling the reference gas unit (30), wherein the cooling device (51, 53) for cooling the reference gas unit (30) is configured as a fan, so that ambient air flows into the housing (68) and heated air flows out of the interior of the housing (68).

2. The measuring device (20) according to claim 1, comprising a second switchable valve (44) arranged in the third gas line (37) downstream of the first switchable valve (42).

3. The measuring device (20) according to claim 2, wherein the first and second switchable valves (42, 44) are solenoid valves.

4. The measuring device (20) according to any one of the preceding claims 2 or 3, comprising a pressure switch (54) arranged in the third gas line (37) between the second valve (44) and the pressure measuring device (56).

5. The measuring device (20) according to claim 1, wherein the measurement chamber is configured to be funnel-shaped.

6. The measuring device (20) according to claim 1, wherein the measuring device (20) further includes an evaluation unit.

7. The measuring device (20) according to claim 1, comprising a further pressure measuring device (34) arranged in the main gas line (28) downstream of the pressure regulator (32).

8. The measuring device (20) according to claim 1, comprising a safety valve (36) arranged in the main gas line (28) downstream of the pressure regulator (32), preferably downstream of the further pressure measuring device (34).

9. A method for determining a hydrocarbon concentration in compressed air by means of a measuring device (20) according to any one of the preceding claims 1 to 8, comprising the method steps:
- introducing the hydrocarbon-containing compressed air into the measuring device (20) and setting a constant through-flow at a pressure range from 3 to 16 bar,
- dividing the hydrocarbon-containing compressed air into a compressed air to be measured and a reference compressed air, wherein the compressed air to be measured is fed directly to a sensor unit (26), and the reference compressed air is first oxidized,
- alternately feeding the compressed air to be measured and the reference compressed air into the sensor unit (26) at a defined interval and determining a pressure of the respective compressed air flowing into the sensor unit (26),
- determining a measurement value from a signal difference between the compressed air to be measured and the reference compressed air, and
- determining a temperature of the respective compressed air leaving the sensor unit (26).

10. The method according to claim 9, wherein the compressed air to be measured is fed longer to the sensor unit (26) by a factor in the range from 1.5 to 5 times, preferably 2 to 4.

11. The method according to claim 9 or 10, wherein a mean value is formed from a plurality of, preferably 5 to 15, detected measurement values.

12. The method according to claim 11, wherein a floating mean value is formed from a plurality of, preferably from 15 to 25, measurement values.

## Revendications

1. Dispositif de mesure (20) destiné à déterminer une concentration en hydrocarbures dans un air comprimé, comprenant un boîtier (68) et les composants suivants agencés dans le boîtier (68):
- une conduite de gaz principale (28) ayant une entrée (31) pour l'air comprimé,
- une première conduite de gaz (38) pour un air comprimé à mesurer, qui dérive de la conduite de gaz principale (28), dans lequel la première conduite de gaz (38) relie la conduite de gaz principale (28) à une première vanne commutable (42),
- une deuxième conduite de gaz (46) pour un air comprimé de référence, qui dérive de la conduite de gaz principale (28), dans lequel la deuxième conduite de gaz (46) relie la conduite de gaz principale (28) à la première vanne commutable (42),
- une troisième conduite de gaz (37) qui relie la première vanne commutable (42) à une unité de capteur (26) et est constituée de cuivre de sorte que de l'air comprimé qui circule dans la troisième conduite de gaz (37) prend la température régnant à l'intérieur du boîtier (68), dans lequel l'unité de capteur (26) présente un détecteur à photoionisation (40) avec une chambre de mesure,
- une unité de gaz de référence (30) qui est disposée dans la deuxième conduite de gaz (46) et qui contient un catalyseur d'oxydation et dans laquelle l'air comprimé de référence est généré à partir de l'air comprimé,
- un régulateur de pression (32) qui est disposé dans la conduite de gaz principale (28) et est destiné à assurer un débit constant de l'air comprimé dans la plage de 3 à 16 bars,
- un dispositif de mesure de pression (56) qui est disposé en amont de l'unité de capteur (26) et est destiné à déterminer une pression de l'air comprimé respectif entrant dans l'unité de capteur (26),
- un capteur de température (62) qui est disposé en aval de l'unité de capteur (26) et est destiné à déterminer une température de l'air comprimé respectif quittant l'unité de capteur (26), et
- un dispositif de refroidissement (51, 53) destiné à refroidir l'unité de gaz de référence (30), dans lequel le dispositif de refroidissement (51, 53) destiné à refroidir l'unité de gaz de référence (30) est conçu en tant que ventilateur de sorte que de l'air ambiant entre dans le boîtier (68) et que de l'air chauffé quitte l'intérieur du boîtier (68).

2. Dispositif de mesure (20) selon la revendication 1, comprenant une deuxième vanne commutable (44) qui est disposée dans la troisième conduite de gaz (37) en aval de la première vanne commutable (42).

3. Dispositif de mesure (20) selon la revendication 2, dans lequel la première et la deuxième vanne commutable (42, 44) est une électrovanne.

4. Dispositif de mesure (20) selon l'une quelconque des revendications précédentes 2 ou 3, comprenant un pressostat (54) qui est disposé dans la troisième conduite de gaz (37) entre la deuxième vanne (44) et le dispositif de mesure de pression (56).

5. Dispositif de mesure (20) selon la revendication 1, dans lequel la chambre de mesure est en forme d'entonnoir.

6. Dispositif de mesure (20) selon la revendication 1, dans lequel le dispositif de mesure (20) comprend en outre une unité d'évaluation.

7. Dispositif de mesure (20) selon la revendication 1, comprenant un autre dispositif de mesure de pression (34) qui est disposé dans la conduite de gaz principale (28) en aval du régulateur de pression (32).

8. Dispositif de mesure (20) selon la revendication 1, comprenant une soupape de sécurité (36) qui est disposée dans la conduite de gaz principale (28) en aval du régulateur de pression (32), de préférence de l'autre dispositif de mesure de pression (34).

9. Procédé de détermination d'une concentration en hydrocarbures dans un air comprimé, au moyen d'un dispositif de mesure (20) selon l'une quelconque des revendications précédentes 1 à 8, comprenant les étapes de procédé consistant à:
- introduire l'air comprimé contenant des hydrocarbures dans le dispositif de mesure (20) et régler un débit constant dans une plage de pression de 3 à 16 bars,
- diviser l'air comprimé contenant des hydrocarbures en un air comprimé à mesurer et un air comprimé de référence, dans lequel l'air comprimé à mesurer est amené directement à une unité de capteur (26) et l'air comprimé de référence est d'abord oxydé,
- introduire l'air comprimé à mesurer et l'air comprimé de référence alternativement dans l'unité de capteur (26) à un intervalle déterminé, et déterminer une pression de l'air comprimé respectif entrant dans l'unité de capteur (26),
- déterminer une valeur mesurée à partir d'une différence de signal entre l'air comprimé à mesurer et l'air comprimé de référence, et
- déterminer une température de l'air comprimé respectif sortant de l'unité de capteur (26).

10. Procédé selon la revendication 9, dans lequel l'air comprimé à mesurer est fourni à l'unité de capteur (26) plus longtemps d'un facteur se situant dans la plage de 1,5 à 5, de préférence de 2 à 4.

11. Procédé selon la revendication 9 ou 10, dans lequel une valeur moyenne est formée à partir d'une pluralité de, de préférence 5 à 15, valeurs mesurées déterminées.

12. Procédé selon la revendication 11, dans lequel une moyenne glissante est formée à partir d'une pluralité de, de préférence 15 à 25, valeurs moyennes.
